(19) Europäisches Patentamt / European Patent Office / Office européen des brevets

(11) **EP 2 490 725 B1**

(12) **EUROPEAN PATENT SPECIFICATION**

(45) Date of publication and mention
of the grant of the patent:
**05.08.2015 Bulletin 2015/32**

(21) Application number: **10825293.3**

(22) Date of filing: **20.10.2010**

(51) Int Cl.:
*A61L 15/42* (2006.01)        *A61F 13/00* (2006.01)
*A61L 15/56* (2006.01)

(86) International application number:
**PCT/SE2010/051130**

(87) International publication number:
**WO 2011/049522 (28.04.2011 Gazette 2011/17)**

(54) **GREEN ARTICLE FOR USE IN WOUND TREATMENT**

GRÜNER ARTIKEL ZUR VERWENDUNG BEI DER WUNDBEHANDLUNG

ARTICLE VERT DESTINÉ À ÊTRE UTILISÉ DANS UN TRAITEMENT DES PLAIES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.10.2009 SE 0901356**

(43) Date of publication of application:
**29.08.2012 Bulletin 2012/35**

(73) Proprietor: **Mölnlycke Health Care AB
402 52 Göteborg (SE)**

(72) Inventor: **NILSSON, Carianne
S-443 92 Lerum (SE)**

(74) Representative: **Valea AB et al
Box 7086
103 87 Stockholm (SE)**

(56) References cited:
**EP-A2- 1 093 824          EP-A2- 1 093 824
US-A- 3 875 937            US-A- 3 961 629
US-A- 4 008 303            US-A- 4 008 303
US-A- 4 938 901            US-A1- 2008 249 494
US-A1- 2009 177 133**

- **DATABASE WPI Week 199806, Derwent
Publications Ltd., London, GB; AN 1998-053077,
XP003027964 & CN 1 138 486 A (CHI MEI MEDICAL
SCI & TECHNOLOGY CO LTD HU) 25 December
1996**

**Description**

TECHNICAL FIELD

The present invention relates to an article for use in wound treatment. The invention also relates to an apparatus for treating wound with negative pressure, said apparatus including a pump for providing said negative pressure to the wound, a wound filler and a sealing film.

BACKGROUND ART

Polymer foam has been used for decades as wound filling material. Normally one has used open-cell foam that can transport body fluids through its network but also perforated closed-cell foam has been suggested.

A highly absorbent hydrophilic polyurethane foam is known from EP 0 335669. This application was filed 1988.

From for instance EP 0620720 A, which was filed with priority from 1991, is known a substantially flat section of open cell polyester foam (Fischer Scientific, Pittsburgh, PA 15219) which is said to be sufficiently large to cover the wound and thus prevent wound overgrowth.

In US patent application 2008208147A, filed in 2007, is mentioned that open-cell polymer foam may be used as wound filler. The examples of polymer foam mentioned in said application are GranuFoam and WhiteFoam.TM dressings marketed by Kinetic Concepts Inc, San Antonio, Tex.

Healing of wound with negative pressure is today an accepted method for treating difficult and with earlier conventional methods slow-healing wounds.

Drainage of, for example, surgical wounds or other weeping wounds with negative pressure is a standard treatment which has been used for decades.

An example of a manual suction pump for this purpose is described in US 3 742 952.

US 3 572 340 describes a pump in the form of an elastically compressible body made of an open-celled foam material, preferably a polyurethane foam, which body also serves as a receptacle for fluid drained from the wound. The pump is said to have a capacity to maintain a negative pressure of 15-80 mmHg for more than 48 hours. A perforated drain is intended to be placed in the wound pocket and is connected to the pump by a tube. A similar device is described in US 4 525 166, in the description of which it is specifically stated that the negative pressure not only drains wound fluid but also draws together the wound edges and stimulates tissue growth and healing of the wound. The two latter publications therefore state that vacuum treatment of wounds stimulates wound healing.

The terms vacuum treatment, treatment with reduced pressure and treatment under negative pressure are used interchangeably in the literature. It should be pointed out that, where these terms are used within this description, treatment at a pressure below normal atmospheric pressure is always meant.

Vacuum treatment of wounds is described in for instance US 4 969 880, US 5 645 081, US 5 636 643, US 6 855 135 and WO 2006/025848 A2. These publications mention the use of polymer foam as wound filling material.

According to its abstract, EP 1 093 824 A2 discloses a bioresorbable polymer, suitable for treatment of wounds, containing a dye.

According to its abstract, US 4 008 303 A discloses polyglycolic acid surgical elements, particularly sutures, which are coloured green to contrast with tissue, blood, and surroundings by extruding while melted the polyglycolic acid and concurrently in an optically homogeneous dispersion, from about 0.03% to 0.5% by weight of 1,4-bis(p-toluidino)-anthraquinone (D&C Green No. 6). A bioresorbable polymer, suitable for treatment of wounds, containing a dye.

Use of polymer foam in wound dressing and as wound filler in connection with negative pressure treatment of wound is thus known since decades as mentioned above.

Polymer foams that exist on the market for use as dressings and as wound filler in connection with negative pressure treatment performs well in many respects but all hitherto known foams for said uses can be improved as will be set forth below in the presentation of the present invention.

GranuFoam, marketed by Kinetic Concepts Inc, San Antonio, Tex. in connection with negative pressure treatment, is a black polyurethane foam. One advantage with black is that this colour differs from other colours in the wound care area. An essential drawback with this black foam is however that a user or a caretaker, such as a nurse, will not be able to see when blood or pus is present in the foam. It is obvious that this in certain situations can be a serious disadvantage.

WhiteFoam.TM also marketed by Kinetic Concepts Inc, San Antonio, Tex. is as the name indicates a white polyurethane foam. It is true that pus and blood will be visible for a user or a caretaker but this is in fact nevertheless a serious drawback. A patient with a white foam as dressing can be scared and worried when blood and/or pus is clearly visible in the white foam. A further disadvantage with white foam is that the foam is not in sharp contrast to other material used in wound. A further more serious risk is that a white foam coloured with blood will not distinguish satisfactorily in a surrounding with blood flow and it is a risk that white foam is left behind in a wound.

The above mentioned problems with existing foam have hitherto not been fully realized or addressed.

The object with the present invention is to find a polymer foam which solves the above mentioned problems with hitherto known polymer foam in the wound care area.

Above has been mentioned the problems with foam used in connection with negative pressure treatment. The problems with hitherto used colour of polymer foam as wound filling material is of course relevant for polymer foam and for other materials that are used in wound treatment in, over, and near the wound area. Another obvious example is gauze which hitherto primarily has been white.

DISCLOSURE OF INVENTION

An article for use in wound treatment is according to the invention characterized in that the article is coloured in a chosen colour, in that the chosen colour is green, and in that the chosen colour in dry state has the following mean values

a lightness factor L* along the black-white axis of between 20-55 according to Test Method T-397,

a chromatic coordinate a* along the red-green axis of between -45 to -20 according to Test Method T-397, and

a chromatic coordinate b* along the yellow-blue axis of between -4.0 to -1.0 according to Test Method T-397.

The inventive concept is that the colour of the article stands out in clear contrast against body tissue and body fluids, such as pus and blood, and also that the article hides the colour of body fluids, such as pus and blood, present in the article and make the presence of body fluids visible through a differentiation in the chosen colour of the article, which differentiation occur when the article in the chosen colour have taken up body fluids, such as pus and/or blood.

According to one embodiment the invention is characterized in that said article is made of cotton, such as gauze.

According to another embodiment the invention is characterized in that said article is made of a non-woven material.

According to one embodiment the invention is characterized in that that said article is made of a netting material.

According to another embodiment the invention is characterized in that said article is made of a polymer foam.

According to an embodiment the invention is characterized in that the polymer foam is an open-cell foam.

According to another embodiment the invention is characterized in that the polymer foam is a closed-cell foam with through holes.

According to a further embodiment the invention is characterized in that said polymer foam is a polyurethane foam, a polyester foam, a polyether foam or a polyvinyl alcohol foam.

According to another embodiment the invention is characterized in that the polymer foam is hydrophobic.

According to another embodiment the invention is characterized in that the polymer foam is hydrophilic or treated with an agent that makes the foam hydrophillic.

According to an embodiment the invention is characterized in that the chosen colour in the dry state has the following mean values

a lightness factor L* along the black-white axis of between 30-45 according to Test Method T-397,

a chromatic coordinate a* of between -40 to -25 according to Test Method T-397, and

a chromatic coordinate b* of between -3.5 to -1.5 according to Test Method T-397.

In an apparatus for treating wound with negative pressure, which apparatus including a pump for providing said negative pressure to the wound, a wound filler and a sealing film the invention is characterized in that said wound filler is made of a material in a chosen colour, in that said chosen colour is green and in dry state has the following mean values

a lightness factor L* along the black-white axis of between 20-55 according to Test Method T-397,

a chromatic coordinate a* along the red-green axis of between -45 to -20 according to Test Method T-397, and

a chromatic coordinate b* along the yellow-blue axis of between -4.0 to -1.0 according to Test Method T-397.

DETAILED DESCRIPTION OF THE INVENTION

The invention will in the following be described in connection with an embodiment where the article is polymer foam for use as a wound filling material. Polymer foam is often used as wound filling material in apparatus for negative pressure treatment of wounds.

When choosing colour of polymer foam for use in the wound area a lot of criteria must be fulfilled for optimal function of the polymer foam as wound filler.

The colour should preferably be in sharp contrast to other material used in the wound area and it should also distinguish from blood when used in a surrounding with heavy blood flow. That is, as mentioned above, a problem with white foam. A further drawback with white foam is that it has a tendency to become yellowish during storage and therefore get an unattractive look.

The problem with black colour of the foam is, as mentioned above, that a user or a caretaker, such as a nurse, will not be able to see when blood or pus is present in the foam. It is obvious that this in certain situations can be a serious disadvantage.

Blue foam has a tendency to fade during storage and therefore get an unattractive look. A drawback with blue foam is also that it will not give a satisfactory contrast to the wound.

We have also found that grey foam has serious drawbacks. Similar to white foam it will not distinguish from blood when wetted with blood and there is a risk that it after use will be left behind in the wound.

We have found that green colour stands out in clear contrast against body tissue and body fluids and is a colour that people feel positive to.

The foam in accordance with the described embodiment of the present invention is coloured in a chosen colour that stands out in clear contrast against body tissue and body fluids, such as pus and blood, and that hide the colour of body fluids present in the foam and make the presence of body fluids visible through a differentiation in the chosen colour of the foam, which differentiation occur when the foam in the chosen colour has taken up body fluids, such as pus and/or blood

We have further found that an optimal function of the appearance of polymer foam in dry as well as wet condition, i.e. wetted with blood or pus, is achieved when the chosen colour is green and when the green polymer foam in dry state has the following mean values

a lightness factor L* along the black-white axis of between 20-55 according to Test Method T-397,

a chromatic coordinate a* along the red-green axis of between -45 to -20 according to Test Method T-397, and

a chromatic coordinate b* along the yellow-blue axis of between -4.0 to -1.0 according to Test Method T-397.

Said method is in accordance with ASTM D 2244-07. The Test Method T-397 makes the measurements according to ASTM D 2244-07 more clear to perform. The Test Method T-397 is described in the enclosed Appendix I.

All measurements according to said method and described below have been made against a white background.

For use in negative pressure treatment of wound the foam is preferably reticulated, i.e. to make a more open cell structure. In negative pressure treatment the foam should promote granulation and allow removal of exudates through the foam. Negative pressure treatment is today a well established method for treatment of difficult and with earlier conventional methods slow-healing wounds. A description of negative pressure treatment can be found in for instance EP 0620720.

An example of a suitable material for the present invention is green polyurethane foam made of polyester polyol with physical characteristics with regards to pore size, tensile strength, hardness and density as the above mentioned GranuFoam.

The foam may be arranged in form of a body of planar form of a certain thickness, such as from 5-30mm, which body during use is cut in accordance with the size of the wound to be treated. Other arrangement is of course possible. For instance the polymer foam may be arranged in form of small balls preferably interconnected with a common string, i.e. as a string of pearls.

Measurements have been performed to determine the absolute colour according to said method T-397 for the above mentioned material as shown in Table 1 below.

Table 1 shows the result for three different batches of green foam material and measurements were performed for two of the batches for non-sterilized and for sterilized foam.

Table 2 below shows for comparison the measurements for blue foam, white foam, black foam, grey foam and for green foam, i.e. green foam in accordance with the invention. Table 2 just shows results for non-sterilized samples in dry state.

Table 3 shows corresponding measurements as in table 2 but for wetted foams, i.e. wetted with synthetic blood and then drained and gently squeezed.

In addition to the test results we have found through visual observation that the presence of blood is clearly visible through a differentiation in the chosen green colour. The green colour darkens when the dry foam is wetted with blood. In wet condition the green foam is darker but it still glimmers in green colour. The presence of blood or pus is clearly indicated by said change in colour differentiation but at the same time the colour of blood and/or pus is hidden.

In contrast one can observe that the white foam is coloured as blood when wetted in blood. This is also the case for grey foam. Blue foam has a tendency to become reddish when wetted with blood and there is a risk that the blue foam when wetted with blood is left behind in a wound.

In contrast to the green foam in accordance with the present invention one cannot see when black foam has been wetted with blood. It is still black without a clear distinction from the dry black foam.

4

Table 1

| Material | Sterile | L* | Std.Dev | a* | Std.Dev | b* | Std.Dev |
|----------|---------|------|---------|-------|---------|------|---------|
| 091808-5B | No | 36.4 | 0.7 | -31.0 | 0.5 | -2.8 | 0.1 |
| 091808-5B | Yes | 35.1 | 1.1 | -29.7 | 1.0 | -2.5 | 0.1 |
| 092626 | No | 35.0 | 1.1 | -29.3 | 0.7 | -3.0 | 0.1 |
| 092626 | Yes | 40.1 | 2.1 | -33.5 | 1.2 | -2.5 | 0.2 |
| 092759 | No | 32.1 | 1.4 | -27.5 | 1.0 | -3.0 | 0.1 |

In the column "Material" in table 1 the different batch numbers for the tested samples are mentioned for said foam material, i.e. polyurethane foam made of polyester polyol, as mentioned above.

The mean values for L*, a* and b* as well as standard deviation for the different batch are given in Table 1 above.

The absolute colour for the green foam according to the invention could be chosen within the limits of:

-    a lightness factor L* along the black-white axis of between 20-55 according to test method T-397,

-    a chromatic coordinate a* along the red-green axis between -45 to -20 according to test method T-397, and

-    a chromatic coordinate b* along the yellow-blue axis of between -4.0 to -1.0 according to test method T-397.

More specifically table 1 indicates that the chosen colour in the dry state has
a lightness factor L* along the black-white axis of between 30-45 according to Test Method T-397,
a chromatic coordinate a* of between -40 to -25 according to Test Method T-397, and
a chromatic coordinate b* of between -3.5 to -1.5 according to Test Method T-397.

Table 2

| Type of foam | | L* | a* | b* |
|--------------|--------|-------|--------|--------|
| Green foam | Mean | 35.00 | -29.30 | -3.00 |
| | Std Dev | 1.10 | 0.70 | 0.10 |
| White foam | Mean | 58.82 | 0.06 | 6.02 |
| | Std Dev | 2.31 | 0.15 | 0.37 |
| Black foam | Mean | 20.11 | 0.08 | -0.30 |
| | Std Dev | 1.10 | 0.08 | 0.06 |
| Blue foam | Mean | 44.71 | -7.74 | -27.03 |
| | Std Dev | 1.90 | 0.58 | 1.56 |
| Grey foam | Mean | 55.66 | -0.32 | 1.65 |
| | Std Dev | 1.09 | 0.07 | 0.40 |

Table 3

| Type of foam + synthetic blood | L* | a* | b* |
|--------------------------------|-------|-------|-------|
| Green foam + synthetic blood | | | |
|    Mean value | 17.51 | -1.36 | -1.17 |
|    Std dev | 2.73 | 2.29 | 0.47 |
| Black foam + synthetic blood | | | |
|    Mean value | 17.91 | 4.01 | 1.12 |
|    Std dev | 1.82 | 0.89 | 0.37 |
| White foam + synthetic blood | | | |
|    Mean value | 26.50 | 27.39 | 9.50 |

(continued)

| Type of foam + synthetic blood | L* | a* | b* |
|---|---|---|---|
| Std dev | 2.36 | 2.64 | 1.52 |
| Blue foam + synthetic blood | | | |
| Mean value | 26.61 | 5.97 | -6.98 |
| Std dev | 3.05 | 2.32 | 2.64 |
| Grey foam + synthetic blood | | | |
| Mean value | 31.16 | 22.90 | 5.39 |
| Std dev | 2.58 | 2.71 | 1.30 |

From table 2 above it is clear that only the green foam fulfils the characteristic features of the shade of colour according to the present invention.

As mentioned above only the chosen green foam works satisfactory in all respects.

In order to illustrate that it is not enough to just choose a green colour twelve different shades of green colour of green polymer foam have been tested. Said twelve different shades go from light green, shade No.1, towards darker and darker green up to shade 12.

Table 4

| Dry | Shade No.1 | | |
|---|---|---|---|
| Measurement | L* | a* | b* |
| 1 | 80.05 | -9.42 | 0.85 |
| 2 | 79.63 | -9.48 | 0.68 |
| 3 | 78.47 | -9.45 | 0.76 |
| Mean value | 79.38 | -9.45 | 0.76 |
| Std dev. | 0.82 | 0.03 | 0.09 |
| | | | |
| Wetted with synthetic blood | Shade No.1 | | |
| Measurement | L* | a* | b* |
| 1 | 30.43 | 34.85 | 11.32 |
| 2 | 27.52 | 34.30 | 10.37 |
| 3 | 31.70 | 35.94 | 12.34 |
| Mean value | 29.88 | 35.03 | 11.34 |
| Std. dev. | 2.14 | 0.83 | 0.99 |
| Dry | Shade No.2 | | |
| Measurement | L* | a* | b* |
| 1 | 76.70 | -17.21 | -2.37 |
| 2 | 76.47 | -17.25 | -2.33 |
| 3 | 76.11 | -17.35 | -2.52 |
| Mean value | 76.43 | -17.27 | -2.41 |
| Std. dev. | 0.30 | 0.07 | 0.10 |
| | | | |

(continued)

| Wetted with synthetic blood | Shade No.2 | | |
|---|---|---|---|
| Measurement | L* | a* | b* |
| 1 | 34.42 | 27.34 | 9.03 |
| 2 | 31.12 | 28.64 | 9.48 |
| 3 | 33.74 | 26.78 | 8.55 |
| Mean value | 33.09 | 27.59 | 9.02 |
| Std. dev. | 1.74 | 0.95 | 0.47 |
| | | | |
| Dry | Shade No.3 | | |
| Measurement | L* | a* | b* |
| 1 | 71.05 | -25.55 | -3.20 |
| 2 | 71.35 | -25.72 | -3.39 |
| 3 | 71.59 | -25.75 | -3.33 |
| Mean value | 71.33 | -25.67 | -3.31 |
| Std. dev. | 0.27 | 0.11 | 0.10 |
| | | | |
| Wetted with synthetic blood | Shade No.3 | | |
| Measurement | L* | a* | b* |
| 1 | 27.49 | 24.04 | 7.69 |
| 2 | 23.53 | 23.74 | 6.94 |
| 3 | 27.08 | 23.30 | 6.92 |
| Mean value | 26.03 | 23.69 | 7.18 |
| Std. dev. | 2.18 | 0.37 | 0.44 |
| | | | |
| Dry | Shade No.4 | | |
| Measurement | L* | a* | b* |
| 1 | 71.27 | -26.52 | -2.19 |
| 2 | 72.02 | -27.16 | -2.54 |
| 3 | 71.09 | -26.58 | -2.69 |
| Mean value | 71.46 | -26.75 | -2.47 |
| Std. dev. | 0.49 | 0.35 | 0.26 |
| | | | |
| Wetted with synthetic blood | Shade No.4 | | |
| Measurement | L* | a* | b* |
| 1 | 28.14 | 23.88 | 7.74 |
| 2 | 29.65 | 19.96 | 6.46 |
| 3 | 26.87 | 21.62 | 6.18 |
| Mean value | 28.22 | 21.82 | 6.79 |
| Std. dev. | 1.39 | 1.97 | 0.83 |

(continued)

| Wetted with synthetic blood | Shade No.4 | | |
|---|---|---|---|
| Measurement | L* | a* | b* |
| | | | |
| Dry | Shade No.5 | | |
| Measurement | L* | a* | b* |
| 1 | 71.00 | -29.21 | -3.36 |
| 2 | 71.97 | -28.80 | -3.19 |
| 3 | 71.68 | -29.71 | -3.34 |
| Mean value | 71.55 | -29.24 | -3.30 |
| Std. dev. | 0.50 | 0.46 | 0.09 |
| | | | |
| Wetted with synthetic blood | Shade No.5 | | |
| Measurement | L* | a* | b* |
| 1 | 30.55 | 20.06 | 6.57 |
| 2 | 25.14 | 22.77 | 6.81 |
| 3 | 28.10 | 19.83 | 5.94 |
| Mean value | 27.93 | 20.89 | 6.44 |
| Std. dev. | 2.71 | 1.64 | 0.45 |
| | | | |
| Dry | Shade No.6 | | |
| Measurement | L* | a* | b* |
| 1 | 67.33 | -34.21 | -2.93 |
| 2 | 67.90 | -33.38 | -3.47 |
| 3 | 67.70 | -33.40 | -3.51 |
| Mean value | 67.64 | -33.66 | -3.30 |
| Std. dev. | 0.29 | 0.47 | 0.32 |
| Wetted with synthetic blood | Shade No.6 | | |
| Measurement | L* | a* | b* |
| 1 | 27.02 | 16.76 | 4.99 |
| 2 | 29.15 | 15.25 | 4.76 |
| 3 | 26.22 | 18.07 | 5.16 |
| Mean value | 27.46 | 16.69 | 4.97 |
| Std. dev. | 1.51 | 1.41 | 0.20 |
| | | | |
| Dry | Shade No.7 | | |
| Measurement | L* | a* | b* |
| 1 | 65.95 | -35.21 | -3.51 |
| 2 | 66.16 | -35.54 | -3.57 |

(continued)

| Dry | Shade No.7 | | |
|---|---|---|---|
| Measurement | L* | a* | b* |
| 3 | 65.69 | -35.27 | -3.55 |
| Mean value | 65.93 | -35.34 | -3.54 |
| Std. dev. | 0.24 | 0.18 | 0.03 |
| Wetted with synthetic blood | Shade No.7 | | |
| Measurement | L* | a* | b* |
| 1 | 22.87 | 16.23 | 4.85 |
| 2 | 22.54 | 16.64 | 4.43 |
| 3 | 24.08 | 16.09 | 4.59 |
| Mean value | 23.16 | 16.32 | 4.62 |
| Std. dev. | 0.81 | 0.29 | 0.21 |
| | | | |
| Dry | Shade No.8 | | |
| Measurement | L* | a* | b* |
| 1 | 65.35 | -36.63 | -3.05 |
| 2 | 63.83 | -36.86 | -3.34 |
| 3 | 64.37 | -36.55 | -3.38 |
| Mean value | 64.52 | -36.68 | -3.26 |
| Std. dev. | 0.77 | 0.16 | 0.18 |
| Wetted with synthetic blood | Shade No.8 | | |
| Measurement | L* | a* | b* |
| 1 | 22.75 | 15.26 | 4.33 |
| 2 | 22.34 | 15.16 | 4.00 |
| 3 | 21.80 | 16.47 | 4.64 |
| Mean value | 22.30 | 15.63 | 4.32 |
| Std. dev. | 0.48 | 0.73 | 0.32 |
| | | | |
| Dry | Shade No.9 | | |
| Measurement | L* | a* | b* |
| 1 | 62.97 | -37.99 | -3.41 |
| 2 | 63.06 | -37.64 | -3.53 |
| 3 | 63.33 | -38.79 | -3.49 |
| Mean value | 63.12 | -38.14 | -3.48 |
| Std. dev. | 0.19 | 0.59 | 0.06 |
| Wetted with synthetic blood | Shade No.9 | | |
| Measurement | L* | a* | b* |
| 1 | 22.34 | 12.11 | 2.69 |
| 2 | 24.20 | 12.76 | 3.55 |

(continued)

| Wetted with synthetic blood | Shade No.9 | | |
|---|---|---|---|
| Measurement | L* | a* | b* |
| 3 | 24.28 | 12.39 | 3.27 |
| Mean value | 23.61 | 12.42 | 3.17 |
| Std. dev. | 1.10 | 0.33 | 0.44 |
| | | | |
| Dry | Shade No.10 | | |
| Measurement | L* | a* | b* |
| 1 | 62.24 | -38.99 | -3.24 |
| 2 | 61.39 | -38.74 | -3.50 |
| 3 | 60.15 | -40.09 | -3.54 |
| Mean value | 61.26 | -39.27 | -3.43 |
| Std. dev. | 1.05 | 0.72 | 0.16 |
| Wetted with synthetic blood | Shade No.10 | | |
| Measurement | L* | a* | b* |
| 1 | 27.15 | 7.17 | 1.82 |
| 2 | 19.86 | 12.28 | 2.74 |
| 3 | 24.50 | 9.47 | 2.35 |
| Mean value | 23.84 | 9.64 | 2.30 |
| Std. dev. | 3.69 | 2.56 | 0.46 |
| | | | |
| Dry | Shade No.11 | | |
| Measurement | L* | a* | b* |
| 1 | 58.47 | -40.59 | -3.39 |
| 2 | 59.73 | -41.81 | -3.35 |
| 3 | 60.27 | -40.10 | -3.30 |
| Mean value | 59.49 | -40.83 | -3.35 |
| Std. dev. | 0.92 | 0.88 | 0.05 |
| Wetted with synthetic blood | Shade No.11 | | |
| Measurement | L* | a* | b* |
| 1 | 21.88 | 6.20 | 1.35 |
| 2 | 20.78 | 7.90 | 1.61 |
| 3 | 24.23 | 6.86 | 1.53 |
| Mean value | 22.30 | 6.99 | 1.50 |
| Std. dev. | 1.76 | 0.86 | 0.13 |
| | | | |
| Dry | Shade No.12 | | |
| Measurement | L* | a* | b* |
| 1 | 51.51 | -41.42 | -3.22 |

(continued)

| Dry | Shade No.12 | | |
|---|---|---|---|
| Measurement | L* | a* | b* |
| 2 | 53.68 | -40.45 | -3.05 |
| 3 | 51.10 | -42.20 | -3.21 |
| Mean value | 52.10 | -41.36 | -3.16 |
| Std. dev. | 1.39 | 0.88 | 0.10 |
| Wetted with synthetic blood | Shade No.12 | | |
| Measurement | L* | a* | b* |
| 1 | 21.51 | 2.32 | 0.07 |
| 2 | 20.51 | 4.30 | 0.32 |
| 3 | 19.63 | 3.15 | 0.09 |
| Mean value | 20.55 | 3.26 | 0.16 |
| Std. dev. | 0.94 | 0.99 | 0.14 |

The results of the measurements shown in table 4 above have been achieved with a slightly modified version of the test method defined in Appendix 1. The only difference is that the number of samples is three instead of five as stipulated by said test method.

The results in table 4 above shows that only shade 12 fulfil the characteristic features of the present invention. It was observed that the colour of shade 12 when wetted with synthetic blood did not change to red. Instead the colour changed to a darker shade which still clearly glimmered in green. The green shade No.12 will therefore be visible also in a bleeding wound. It was observed that the shades 1-11 when wetted with synthetic blood were coloured red. The shade of the red colour darkens more and more from shade 1 to 11.

The test results shown in table 4 above are for different shades of colour of dense (closed) polymer foam. The twelve tested samples were non-reticulated polyester based PUR foam. All samples had a size of $10 \times 10$cm. The thickness was about 2cm.

Further tests have been performed with different grades of green colour for open reticulated foam. Two different shades of green open reticulated foam have been tested.

One of the foam is light green, open reticulated polyether based PUR foam. Said open foam had a thickness of 3cm.

The other foam is also open reticulated polyester based PUR foam but with a darker shade of green. The thickness of the tested sample of this open foam was also about 3cm.

Table 5

| Open reticulated-foam, light green Dry | | | |
|---|---|---|---|
| Measurement | L* | a* | b* |
| 1 | 35.08 | -23.85 | 4.55 |
| 2 | 35.00 | -24.04 | 4.65 |
| 3 | 32.99 | -22.42 | 6.26 |
| 4 | 36.08 | -24.62 | 4.08 |
| 5 | 34.28 | -24.08 | 5.47 |
| Mean value | 34.69 | -23.80 | 5.00 |
| Std. dev. | 1.14 | 0.82 | 0.86 |

It is clear from the measurement results that this shade of open reticulated green foam does not fulfil the characteristic features of the present invention.

Table 6

| Open reticulated foam, light green Wetted with synthetic blood | | | |
|---|---|---|---|
| Measurement | L* | a* | b* |
| 1 | 14.44 | 5.10 | 1.84 |
| 2 | 15.92 | 3.13 | 1.55 |
| 3 | 16.21 | 2.78 | 2.17 |
| 4 | 16.18 | 1.99 | 1.06 |
| 5 | 15.28 | 3.81 | 1.76 |
| Mean value | 15.61 | 3.36 | 1.68 |
| Std. dev. | 0.75 | 1.17 | 0.41 |

It was observed that the colour of the foam when wetted with synthetic blood changed from green to red.

Table 7

| Open reticulated foam, darker shade of green Dry | | | |
|---|---|---|---|
| Measurement | L* | a* | b* |
| 1 | 32.94 | -28.06 | -2.29 |
| 2 | 32.89 | -28.07 | -2.27 |
| 3 | 34.72 | -30.18 | -2.39 |
| 4 | 31.84 | -26.90 | -2.28 |
| 5 | 32.35 | -29.05 | -2.20 |
| Mean value | 32.95 | -28.45 | -2.29 |
| Std. dev. | 1.09 | 1.23 | 0.07 |

It is clear from table 6 above that the open reticulated green foam of a darker shade of green compared with the light green foam fulfils all the characteristic features of the claimed invention.

Table 8

| Open reticulated foam, darker shade of green Wetted with synthetic blood | | | |
|---|---|---|---|
| Measurement | L* | a* | b* |
| 1 | 12.82 | 1.01 | -0.74 |
| 2 | 14.88 | -1.05 | -1.68 |
| 3 | 13.92 | 0.59 | -0.91 |
| 4 | 15.27 | -0.93 | -1.23 |
| 5 | 15.83 | -1.89 | -1.44 |
| Mean value | 14.54 | -0.45 | -1.20 |
| Std. dev. | 1.19 | 1.21 | 0.38 |

It was observed that the colour of the foam, which in dry state has darker shade of green, when wetted with synthetic blood did not change to red. Instead the colour changed to a darker shade which still clearly glimmered in green. The green foam with darker shade of green will therefore be visible also in a bleeding wound.

In contrast the green foam with the test result as stated in table 5 and 6 above will not when wetted with blood be visible in a bleeding wound.

The tests described above have been performed with articles made of polymer foam. The present invention is however not limited to polymer foam. The important features are that the colour of the material is green and that the chosen colour is defined as set forth in the following claims.

The present invention covers all materials that are used in and around a wound.

Examples of materials are:

- non-woven,
- a netting material, for instance as defined in EP 0261167 A1 or
- cotton, such as gauze.

All of said materials can be used as wound dressing or as wound filling material for instance in connection with negative pressure treatment.

On the market exists green ribbon gauze with the trade name Sorbact Ribbon Gauze. Measurements according to Test Method T-397 have been performed for dry Sorbact Ribbon Gauze. The results are shown in table 9 below.

Measurements according to Test Method T-397 have also been performed for Sorbact Ribbon Gauze after wetting the gauze in synthetic blood. The results are shown in table 10 below.

On the market exists green compress with the trade name Sorbact Compress. Measurements according to Test Method T-397 have been performed for dry Sorbact Compress. The results are shown in table 11 below.

Measurements according to Test Method T-397 have also been performed for Sorbact Compress after wetting the gauze in synthetic blood. The results are shown in table 12 below.

Table 9

| Sorbact ribbon gauze (single layer) Dry | | | |
|---|---|---|---|
| Measurement | L* | a* | b* |
| 1 | 73.85 | -28.03 | -4.40 |
| 2 | 72.72 | -28.86 | -4.48 |
| 3 | 71.85 | -30.28 | -4.54 |
| 4 | 71.62 | -30.04 | -4.58 |
| 5 | 70.21 | -32.16 | -4.32 |
| Mean value | 72.05 | -29.87 | -4.46 |
| Std. dev. | 1.35 | 1.57 | 0.11 |

It is clear from the measurement results that this green gauze does not fulfil the characteristic features of the present invention.

Table 10

| Sorbact ribbon gauze (single layer) Wetted with synthetic blood | | | |
|---|---|---|---|
| Measurement | L* | a* | b* |
| 1 | 41.24 | 18.04 | 4.42 |
| 2 | 34.79 | 20.98 | 5.86 |
| 3 | 34.10 | 21.70 | 6.20 |

(continued)

| Sorbact ribbon gauze (single layer) Wetted with synthetic blood | | | |
|---|---|---|---|
| Measurement | L* | a* | b* |
| 4 | 36.76 | 20.94 | 5.92 |
| 5 | 38.77 | 20.23 | 5.28 |
| Mean value | 37.13 | 20.38 | 5.54 |
| Std. dev. | 2.93 | 1.41 | 0.71 |

It was observed that the colour of the green gauze when wetted with synthetic blood changed from green to red. The gauze when wetted with blood will not be visible in a bleeding wound.

Table 11

| Sorbact compress (single layer) Dry | | | |
|---|---|---|---|
| Measurement | L* | a* | b* |
| 1 | 80.32 | -18.68 | -2.05 |
| 2 | 80.34 | -18.32 | -2.22 |
| 3 | 80.12 | -18.14 | -2.23 |
| 4 | 80.44 | -18.09 | -2.24 |
| 5 | 80.95 | -17.54 | -2.09 |
| Mean value | 80.43 | -18.15 | -2.17 |
| Std. dev. | 0.31 | 0.41 | 0.09 |

It is clear from the measurement results that this green compress does not fulfil the characteristic features of the present invention.

Table 12

| Sorbact compress (single layer) Wetted with synthetic blood | | | |
|---|---|---|---|
| Measurement | L* | a* | b* |
| 1 | 47.65 | 15.31 | 4.90 |
| 2 | 48.98 | 14.28 | 4.34 |
| 3 | 56.66 | 10.57 | 2.55 |
| 4 | 50.48 | 13.40 | 4.09 |
| 5 | 54.22 | 11.45 | 3.12 |
| Mean value | 51.60 | 13.00 | 3.80 |
| Std. dev. | 3.75 | 1.96 | 0.95 |

It was observed that the colour of the green gauze when wetted with synthetic blood changed from green to red. The green gauze when wetted with blood will not be visible in a bleeding wound.

The test results on tables 9-12 confirm that it is not enough to choose a green colour to achieve the benefits of the present invention. One also has to choose shade of green colour as defined according to the present invention.

The article according to the invention is not limited to be used as a wound filling material. The invention covers all type of medical articles that are applied in, over and around a wound.

APPENDIX 1

Test Method T-397

DETERMINATION OF COLOUR AND OPACITY

**1**  REFERENCE METHOD

ASTM D 2244 - 07

**2**  PURPOSE

To determine absolute colour and/or opacity

**3**  FIELD OF APPLICATION

All kind of materials

**4**  DEFINITIONS

The result for *colour measurement* reported according to L*a*b* colour system
(CIE 1976) where:
L* = lightness factor, black-white axis (0 black/100 white)
a* = chromatic coordinate, red-green axis (+ red / - green)
b* = chromatic coordinate, yellow-blue axis (+ yellow / - blue)

The result for *opacity* is reported according to the Y-x-y colour system (CIE 1931) where:
Y = % light reflectance
x and y = chromaticity coordinates

**5**  PRINCIPLE

Illumination is provided by a pulsed lamp (CIE Illuminent D 65). The light illuminates the sample to be measured
and reflects on to the photocells which convert the light into chromatic and lightness data.

**6**  PREPARATION OF TEST PIECES

| | |
|---|---|
| Material required- | At least 10 x 10 cm of sample material or 5 whole products |
| Number of measurements | 5 |
| Preparation | Not applicable |
| Conditioning | Not applicable - but important that both the calibration and the measurements are done in the same climate condition. |

**7**  EQUIPMENT

Minolta Chroma Meter CR 300
For opacity measurements, at least five sheets of respectively; black printing paper and white copy paper

**8**  PROCEDURE

(continued)

Calibrate the instrument according to Calibration Instruction and make sure you handle the instrument correctly.

*Calibration Instruction:*

1. Press Calibrate. Wait for about 5 seconds until "Set Cal data display →Measure" has changed to "CAL ch00Y-x-y" (calibrating channel 00). If the displayed colour space is not Y-x-y, press Colour Space Select repeatedly until that is the case. See that the displayed numbers for Y-x-y correspond to the listed data inside the cover of the white calibration plate. If not - see the instruction manual!

2. Place tip of measuring head flat at the center of the white calibration plate. Press Measure or measuring head's measuring button after ready lamp on measuring head lights. Three measurements will automatically be taken and do not move the measuring head until the measurements are ready.

3. After about 5 seconds "CAL" will be replaced by "END" in the display.
Calibration is now complete.

*Colour measurement:*

1. Press Colour Space Select repeatedly until L*a*b*-space is displayed.

2. Place tip of the measuring head flat to the surface of the sample and press Measure or press the button of the measuring head after ready lamp on measuring head is lit.

3. Carry out 5 approved measurements on each sample.

4. Press the Statistic button followed by Enter to receive the mean and the standard deviation.

5. Choose a new page for the next sample by pressing the Page button followed by Enter.

6. Repeat step 2 -5 for the next sample.

*Opacity:*

1. Press Colour Space Select repeatedly until Y-x-y-space is displayed.

2. To obtain an opacity number, two different measurements will have to be conducted. One with the specimens on top of a black surface (or right in the air), to get the $Y_{black}$, and one on top of a white surface, to get the $Y_{white}$.

3. First, carry out 5 measurements of the sample placed on top of a black surface (five sheets of black printing paper) and then press the Statistic button followed by Enter to receive the mean and the standard deviation for $Y_{black}$.

4. Thereafter, carry out 5 measurements at the same spots of the sample, as before, but placed on top of a white surface (five sheets of white copy paper) and then press the Statistic button followed by Enter to receive the mean and the standard deviation for $Y_{white}$.
Note; Make sure that the corresponding $Y_{black}$ and $Y_{white}$ are made on the same spot of the sample.

5. Choose a new page for the following sample by pressing the Page button followed by Enter.

6. Repeat step 2-5 for the next sample.

**9** CALCULATIONS

Opacity number:

$$O = Y_{black}/Y_{white} * 100$$

(continued)

**10** REPORTING
Method and if any deviations
Material and origin
Sample designation

Number of measurements
Mean value and standard deviation of L*, a* and b* for colour measurements
Mean value and standard deviation of $Y_{black}$ and $Y_{white}$ and the opacity number, O, for opacity measurements

## Claims

1. Article for use in wound treatment, wherein the article is coloured in a chosen colour, the chosen colour is green, **characterized in that** the chosen colour in dry state has
   a lightness factor L* along the black-white axis of between 20-55 according to Test Method T-397,
   a chromatic coordinate a* along the red-green axis of between -45 to -20 according to Test Method T-397, and
   a chromatic coordinate b* along the yellow-blue axis of between -4.0 to -1.0 according to Test Method T-397.

2. Article according to claim 1, **characterized in that** said article is made of cotton, such as gauze.

3. Article according to claim 1, **characterized in** said article is made of a non-woven material.

4. Article according to claim 1, **characterized in that** said article is made of a netting material.

5. Article according to claim 1, **characterized in that** said article is a polymer foam.

6. Article according to claim 5, **characterized in that** the polymer foam is an open-cell foam.

7. Article according to claim 5, **characterized in that** the polymer foam is a closed-cell foam with through holes.

8. Article according to any of claims 5-7, **characterized in that** said polymer foam is a polyurethane foam, a polyester foam, a polyether foam or a polyvinyl alcohol foam.

9. Article according to any of claims 5-8, **characterized in that** the polymer foam is hydrophobic.

10. Article according to any of claims 5-8, **characterized in that** the polymer foam is hydrophilic or treated with an agent that makes the foam hydrophilic.

11. Article according to any of the preceding claims, **characterized in that** the chosen colour in the dry state has the following mean values
    a lightness factor L* along the black-white axis of between 30-45 according to Test Method T-397,
    a chromatic coordinate a* of between -40 to -25 according to Test Method T-397, and
    a chromatic coordinate b* of between -3.5 to -1.5 according to Test Method T-397.

12. Apparatus for treating wound with negative pressure, said apparatus including a pump for providing said negative pressure to the wound, a wound filler and a sealing film, wherein said wound filler is made of a material in a chosen colour, said chosen colour is green, **characterized in that** the chosen colour in dry state has the following mean values
    a lightness factor L* along the black-white axis of between 20-55 according to Test Method T-397,
    a chromatic coordinate a* along the red-green axis of between -45 to -20 according to Test Method T-397, and
    a chromatic coordinate b* along the yellow-blue axis of between -4.0 to -1.0 according to Test Method T-397.

**Patentansprüche**

1. Gegenstand zur Verwendung in der Wundbehandlung, wobei der Gegenstand in einer gewählten Farbe gefärbt ist, die gewählte Farbe grün ist, **dadurch gekennzeichnet, dass** die gewählte Farbe im trockenen Zustand einen Helligkeitsfaktor L* entlang der Schwarz-Weiß-Achse von zwischen 20-55 gemäß dem Testverfahren T-397, eine chromatische Koordinate a* entlang der Rot-Grün-Achse von zwischen -45 bis -20 gemäß dem Testverfahren T-397 sowie eine chromatische Koordinate b* entlang der Gelb-Blau-Achse von zwischen -4,0 bis -1,0 gemäß dem Testverfahren T-397 aufweist.

2. Gegenstand gemäß Anspruch 1, **dadurch gekennzeichnet, dass** der Gegenstand aus Baumwolle, wie zum Beispiel Verbandmull, hergestellt ist.

3. Gegenstand gemäß Anspruch 1, **dadurch gekennzeichnet, dass** der Gegenstand aus einem Vliesmaterial hergestellt ist.

4. Gegenstand gemäß Anspruch 1, **dadurch gekennzeichnet, dass** der Gegenstand aus einem Netzmaterial hergestellt ist.

5. Gegenstand gemäß Anspruch 1, **dadurch gekennzeichnet, dass** der Gegenstand ein Polymerschaum ist.

6. Gegenstand gemäß Anspruch 5, **dadurch gekennzeichnet, dass** der Polymerschaum ein offenzelliger Schaum ist.

7. Gegenstand gemäß Anspruch 5, **dadurch gekennzeichnet, dass** der Polymerschaum ein geschlossenzelliger Schaum mit Durchgangslöchern ist.

8. Gegenstand gemäß einem der Ansprüche 5-7, **dadurch gekennzeichnet, dass** der Polymerschaum ein Polyurethanschaum, ein Polyesterschaum, ein Polyetherschaum oder ein Polyvinylalkoholschaum ist.

9. Gegenstand gemäß einem der Ansprüche 5-8, **dadurch gekennzeichnet, dass** der Polymerschaum hydrophob ist.

10. Gegenstand gemäß einem der Ansprüche 5-8, **dadurch gekennzeichnet, dass** der Polymerschaum hydrophil ist oder mit einem Mittel behandelt ist, welches den Schaum hydrophil macht.

11. Gegenstand gemäß einem der vorangehenden Ansprüche, **dadurch gekennzeichnet, dass** die gewählte Farbe in trockenem Zustand die folgenden Mittelwerte aufweist einen Helligkeitsfaktor L* entlang der Schwarz-Weiß-Achse von zwischen 30-45 gemäß dem Testverfahren T-397, eine chromatische Koordinate a* von zwischen -40 bis -25 gemäß dem Testverfahren T-397 sowie eine chromatische Koordinate b* von zwischen -3,5 bis -1,5 gemäß dem Testverfahren T-397.

12. Vorrichtung zur Wundbehandlung mit negativem Druck, wobei die Vorrichtung eine Pumpe zur Bereitstellung des negativen Drucks an der Wunde, ein Wundfüllmittel und einen Verschlussfilm umfasst, wobei das Wundfüllmittel aus einem Material in einer gewählten Farbe hergestellt ist, die gewählte Farbe grün ist, **dadurch gekennzeichnet, dass** die gewählte Farbe in trockenem Zustand die folgenden Mittelwerte aufweist einen Helligkeitsfaktor L* entlang der Schwarz-Weiß-Achse von zwischen 20-55 gemäß dem Testverfahren T-397, eine chromatische Koordinate a* entlang der Rot-Grün-Achse von zwischen -45 bis -20 gemäß dem Testverfahren T-397 sowie eine chromatische Koordinate b* entlang der Gelb-Blau-Achse von zwischen -4,0 bis -1,0 gemäß dem Testverfahren T-397.

**Revendications**

1. Article pour son utilisation dans le traitement des plaies, dans lequel l'article est coloré en une couleur choisie, la couleur choisie est le vert, **caractérisé en ce que** la couleur choisie dans un état sec a un facteur de légèreté L* le long de l'axe noir-blanc compris entre 20 et 55 selon un procédé de test T-397, une coordonnée chromatique a* le long de l'axe rouge-vert comprise entre -45 et -20 selon un procédé de test T-397, et une coordonnée chromatique b* le long de l'axe jaune-bleu comprise entre -4,0 et - 1,0 selon un procédé de test T-397.

**2.** Article selon la revendication 1, **caractérisé en ce que** ledit article est en coton, par exemple en gaze.

**3.** Article selon la revendication 1, **caractérisé en ce que** ledit article est en un matériau non tissé.

**4.** Article selon la revendication 1, **caractérisé en ce que** ledit article est en un matériau de filet.

**5.** Article selon la revendication 1, **caractérisé en ce que** ledit article est une mousse polymère.

**6.** Article selon la revendication 5, **caractérisé en ce que** la mousse polymère est une mousse à cellules ouvertes.

**7.** Article selon la revendication 5, **caractérisé en ce que** la mousse polymère est une mousse à cellules fermées avec des trous débouchants.

**8.** Article selon l'une quelconque des revendications 5 à 7, **caractérisé en ce que** ladite mousse polymère est une mousse polyuréthane, une mousse polyester, une mousse polyéther ou une mousse alcool polyvinylique.

**9.** Article selon l'une quelconque des revendications 5 à 8, **caractérisé en ce que** la mousse polymère est hydrophobe.

**10.** Article selon l'une quelconque des revendications 5 à 8, **caractérisé en ce que** la mousse polymère est hydrophile ou traitée avec un agent qui rend la mousse hydrophile.

**11.** Article selon l'une quelconque des revendications précédentes, **caractérisé en ce que** la couleur choisie à l'état sec a les valeurs moyennes suivantes :

un facteur de légèreté L* le long de l'axe noir-blanc compris entre 30 et 45 selon un procédé de test T-397,
une coordonnée chromatique a* comprise entre -40 et -25 selon un procédé de test T-397, et
une coordonnée chromatique b* comprise entre -3,5 et -1,5 selon un procédé de test T-397.

**12.** Appareil de traitement des plaies avec une pression négative, ledit appareil comprenant une pompe pour fournir ladite pression négative à la plaie, une substance de comblement de plaies et un film d'étanchéité, dans lequel ladite substance de comblement est en un matériau dans une couleur choisie, ladite couleur choisie est le vert, **caractérisé en ce que** la couleur choisie à l'état sec a les valeurs moyennes suivantes :

un facteur de légèreté L* le long de l'axe noir-blanc compris entre 20 et 55 selon un procédé de test T-397,
une coordonnée chromatique a* le long de l'axe rouge-vert comprise entre -45 et -20 selon un procédé de test T-397, et
une coordonnée chromatique b* le long de l'axe jaune-bleu comprise entre -4,0 et - 1,0 selon un procédé de test T-397.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0335669 A
- EP 0620720 A
- US 2008208147 A
- US 3742952 A
- US 3572340 A
- US 4525166 A
- US 4969880 A
- US 5645081 A
- US 5636643 A
- US 6855135 B
- WO 2006025848 A2
- EP 1093824 A2
- US 4008303 A
- EP 0261167 A1